# EUROPEAN PATENT APPLICATION

(11) **EP 3 190 532 A1**
(43) Date of publication of application: **12.07.2017**
(21) Application number: 17150547.2
(22) Date of filing: 06.01.2017
(51) Int. Cl.: G06F 19/00

(54) **CONTROLLER FOR TRACKING PATIENT CONSUMABLES**

(30) Priority: 07.01.2016 US 201662275940 P
(71) Applicant: Hill-Rom Services, Inc., Batesville, IN 47006-9167 (US)
(72) Inventor: OWSLEY, Clay Gerome, Batesville, IN 47006-9167 (US); RAJANI, Umesh Jairamdas, Batesville, IN 47006-9167 (US); LUPTON, Jonathan K., Batesville, IN 47006-9167 (US); RUDE, Jared, Batesville, IN 47006-9167 (US); ROSS, Robert, Batesville, IN 47006-9167 (US); ZHOU, Fengshuan, Batesville, IN 47006-9167 (US); HIXON, Nathaniel W., Batesville, IN 47006-9167 (US)
(74) Representative: Findlay, Alice Rosemary

(57) **Abstract**

A controller of a patient support apparatus is disclosed. The controller includes a processor, an electro-magnetic reader configured to transmit data to the processor, a user input configured to transmit a signal to the processor responsive to an activation of the user interface by a user, and memory. A plurality of instructions are stored in the memory and executed by the processor.

## Description

The present disclosure relates to patient support apparatuses, such as hospital beds, for example, which include controllers operable to communicate wirelessly. More specifically, the present disclosure relates to patient support apparatuses having controllers that communicate with patient consumables and are operable to act on data received from the patient consumables.

Some patient support apparatuses, such as hospital beds, have become more sophisticated including the implementation of communications networks between various modules of the patient support apparatus. In a hospital setting, the identification and location of a particular patient is often associated with a particular patient support apparatus. A unique identifier which identifies a particular patient may be stored with the patient support apparatus or with a hospital information system via a hospital network in communication with the patient support apparatus. The patient is associated with the appropriate patient support apparatus to allow the hospital information system to track the patient and maintain the patient's electronic record.

With the advent and proliferation of electronic medical records systems, caregivers are expected to chart patient data in real time. It is important to maintain adequate records of patient care received so that a physician can interpret, from a distance, data in the electronic medical records system to thereby modify a patient's treatment plan. Caregivers are challenged to provide continuous care and attention to the patient, while also fulfilling the expectations of real time charting.

The present application discloses one or more of the following features alone or in any combination.

According to the present discloses a controller of a patient support apparatus comprises a processor configured to communicate with a hospital network, an electro-magnetic reader configured to receive an electro-magnetic signal from a patient consumable and to transmit data to the processor, and a memory having stored therein a plurality of instructions that when executed by the processor cause the controller to receive, automatically, data from the electro-magnetic reader indicative of unique patient-consumable identification information of a patient consumable being administered to a patient associated with the patient support apparatus in response to the electro-magnetic reader receiving the electro-magnetic signal from the patient consumable, and transmit data and instructions to the hospital network to generate a record indicative that the patient consumable has been expended , the data including the associated unique patient-consumable identification information

In some embodiments the instructions transmitted to the hospital network cause the hospital network to update an inventory database stored on the hospital network to indicate that the patient consumable has been expended.

In some embodiments the plurality of instructions further cause the controller to receive unique identification information of a patient associated with the patient support apparatus from at least one of a user input and the hospital network, associate the unique patient-consumable identification information with the unique identification information of the patient, and transmit data and instructions to the hospital network to generate a record that the patient consumable has been administered to the patient, the data also including the unique identification of the patient.

In some embodiments the plurality of instructions further cause the controller to receive unique identification information of a patient associated with the patient support apparatus from at least one of a user input and the hospital network.

The plurality of instructions may further cause the controller to prompt the caregiver to verify, through the user input, unique identification information of the patient to whom the patient consumable is being administered.

The plurality of instructions may further cause the controller to prompt the caregiver to indicate whether the patient consumable was digested by the patient. The record may indicate that the patient consumable was digested by the patient.

In some embodiments the plurality of instructions further cause the controller to prompt the caregiver to indicate, through a user input, whether the patient consumable is to be billed to the patient, and generate, automatically, a record indicative that the patient consumable is to be billed to the patient if the caregiver indicated that the patient consumable is to be billed to the patient.

The user input may be a touch screen and the plurality of instructions cause the user input display (i) a first touch-to-accept option to bill the patient consumable to the patient and (ii) a second touch-to-accept option not to bill the patient consumable to the patient to prompt the caregiver to indicate whether the patient consumable is to be billed to the patient. The plurality of instructions stored in the memory may further cause the controller to display information relating to the patient consumable on the touch screen.

In some embodiments the electro-magnetic reader is configured to receive an electro-magnetic signal from the patient consumable without the patient consumable being within line-of-sight of the electro-magnetic reader.

In some embodiments the electro-magnetic reader includes a RFID reader configured to receive an electro-magnetic signal from an RFID tag attached to the patient consumable.

In some embodiments the electro-magnetic reader includes a barcode reader configured to receive an electro-magnetic signal from a barcode attached to the patient consumable.

The controller may include a user input configured to transmit a signal to the processor responsive to an activation of the user input by a caregiver.

The invention will now be further described by way of example with reference to the accompanying drawings, in which:
FIG. 1 is a patient support apparatus including an illustrative controller configured to detect a patient consumable being administered to a patient by a caregiver, prompt the caregiver to indicate if the patient consumable should be billed to the patient, and generate a record of the patient consumable if the caregiver indicates that the patient consumable should be billed to the patient;
FIG. 2 is a diagrammatic representation of the structure of a system including the patient support apparatus and patient consumable of FIG. 1;
FIG. 3 is a screen shot of a an illustrative graphical user interface included in the patient support apparatus of FIG. 1 displaying a prompt for the caregiver to indicate whether the patient should be billed for the patient consumable; and
FIG. 4 is a flow chart describing an illustrative operational process of the controller of the patient support apparatus of FIG. 1.

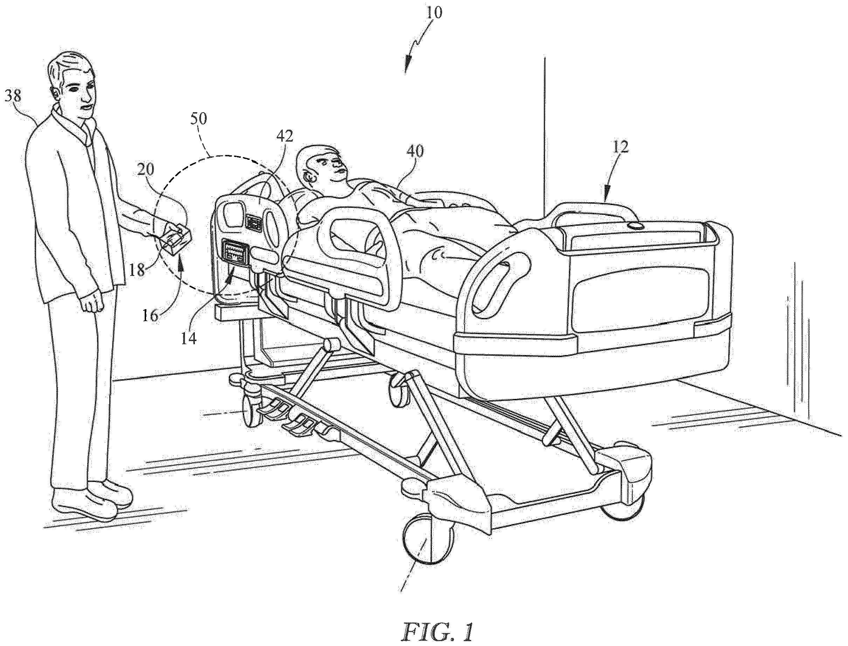

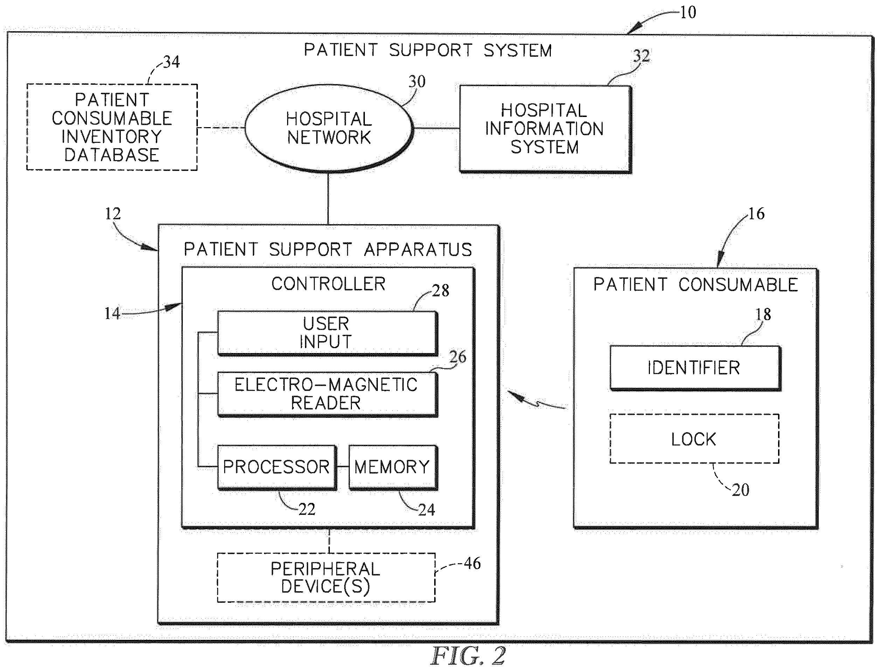

An illustrative system 10 for gathering information from patient consumables 16 and transferring information to a hospital information system 32 is shown in FIGS. 1 and 2. The illustrative system 10 includes a patient support apparatus 12, a patient consumable 16, and a hospital information system 32 as shown in FIGS. 1 and 2. The patient support apparatus 12 includes a controller 14 operable to communicate with the patient consumable 16 and with the hospital information system 32 as suggested in FIG. 2.

The patient consumable 16 may include one or more of any number of consumables. An illustrative and non-exclusive list of examples of patient consumables 16 includes medication, treatments, wound dressing, personal respiratory therapy devices, intravenous (IV) sets, ports, bandages, garments, gowns, linens, injections, gastric tubes, sensors, sequential compression garments, catheters, thermometer covers, blood pressure cuffs, wound packing, central lines, arterial lines, temporary pacemaker wires, epidural catheters, subdural catheters, endotracheal tubes, chest tubes, surgical drains, implantable devices, shunts, and other disposable patient care consumables. In the illustrative embodiment, the patient consumable 16 includes medication 16 as shown in FIG. 1.

In illustrative embodiments, the patient consumable 16 includes an identifier 18 as shown in FIGS. 1 and 2. The controller 14 is configured to receive information from the identifier 18. The information received from the identifier 18 includes unique patient-consumable identification information relating to the patient consumable 16. The controller 14 identifies the patient consumable 16 being administered to the patient 40 in response to receiving information from the identifier 18. In some embodiments, the identifier 18 is coupled to the patient consumable 16. In some embodiments, the identifier 18 is coupled to a container housing the patient consumable 16.

In some embodiments, the identifier 18 includes an electro-magnetic identifier 18 as shown in FIG. 1. In the illustrative embodiment, the identifier 18 includes a radiofrequency identification (RFID) tag 18. The controller 14 is configured to receive a signal from the RFID tag 18 and to identify the unique patient consumable 16 using information in the signal. The signal allows the controller 14 to receive the unique patient-consumable information without the controller 14 being within line-of-sight of the RFID tag 18. In other embodiments, the identifier 18 includes a barcode, a magnetic stripe, or any other alternative machine readable identifier.

In some embodiments, one or more identifiers 18 may be located in a catalog. As an example, the identifier 18 may be presented on a catalog of barcodes may be located near the controller 14. The controller 14 is configured to read individual identifiers 18 in the catalog with the assistance of the caregiver 38. As such, an identifier 18 may not be coupled to each patient consumable 16.

In some embodiments, the patient consumable 16 and/or a container housing the patient consumable 16 includes a lock 20 as shown in FIG. 2. The lock 20 is arranged to block access to the patient consumable 16 so that the patient consumable 16 is blocked from being administered to the patient 40. In some embodiments, the controller 14 determines if the patient consumable 16 may be administered to the patient 40 by comparing the unique patient-consumable identification to information received from the hospital information system 32 via a hospital network 30. If the controller 14 determines the patient consumable 16 may be administered to the patient 40, the controller 14 unlocks the lock 20 to allow access to the patient consumable 16. In some embodiments, the controller 14 determines if the caregiver 38 is authorized to administer the patient consumable 16 to the patient 40 by comparing unique caregiver identification information to information received from the hospital information system 32 via the hospital network 30. If the controller 14 determines the caregiver 38 may administer the patient consumable 16 to the patient 40, the controller 14 unlocks the lock 20 to allow access to the patient consumable 16.

The controller 14 is configured to make a record at the point of care as shown in FIG. 1. In the illustrative embodiment, the controller 14 is included in a side rail 42 of the patient support apparatus 12. Illustratively, the controller 14 is configured to automatically detect and identify the patient consumable 16 in response to the identifier 18 coupled to the patient consumable 16 entering a detection range 50 of the controller 14 as suggested in FIG. 1.

In the illustrative embodiment, unique identification and location information of each particular patient 40 is associated with a particular patient support apparatus 12. The unique identifier which identifies a particular patient 40 may be stored with the controller 14 or with the hospital information system 32 via the hospital network 30 in communication with the patient support apparatus 12. The patient 40 is associated with the appropriate patient support apparatus 12 to allow the hospital information system 32 to track the patient 40 and maintain the patient's electronic record.

The controller 14 includes a processor 22 and a memory 24 as shown in FIG. 2. The processor 22 is operable to use instructions stored in the memory 24 to receive information relating to the patient 40 and the patient consumable 16 and to communicate with the hospital network 30. The data includes associated unique patient-consumable identification information and unique identification information of the patient 40.

In the illustrative embodiment, the controller 14 further includes an electro-magnetic reader 26 and a user input 28 connected to the processor 22 as shown in FIG. 2. The electro-magnetic reader 26 is configured to receive an electro-magnetic signal from the identifiers 18 with or without the identifier 18 being within line-of-sight of the electro-magnetic reader and to transmit data to the processor 22. The user input 28 is configured to receive input from the caregiver 38 and to transmit a signal to the processor 22 responsive to an activation of the user input 28 by the caregiver 38.

The electro-magnetic reader 26 is configured to detect the identifier 18 of the patient consumable 16, identify the patient consumable 16, and transmit data such as, for example, data indicative of the unique patient-consumable identification information to the processor 22. In the illustrative embodiment, the electro-magnetic reader 26 continuously scans for an identifier 18. In some embodiments, the electro-magnetic reader 26 periodically scans for an identifier 18. In some embodiments, the electro-magnetic reader 26 scans for an identifier 18 in response to receiving a command to scan from the caregiver 38.

In the illustrative embodiment, the electro-magnetic reader 26 is an radiofrequency identification (RFID) reader 26 configured to receive a radio frequency from an RFID tag 18 coupled to the patient consumable 16 as shown in FIG. 1. In other embodiments, the electro-magnetic reader 26 is a barcode reader, a magnetic stripe reader, or any other suitable alternative. In some embodiments, the electro-magnetic reader 26 is omitted and the unique patient-consumable identification information of the patient consumable 16 is entered into the user input 28 and transmitted to the processor 22.

In the illustrative embodiment, the electro-magnetic reader 26 is configured to identify identifiers associated with the patient 40 and the caregiver 38. A unique patient identifier may be included in a patient wristband secured to the patient 40. The patient wristband may be scanned by the controller 14 when the patient 40 is initially assigned to the patient support apparatus 12 and/or when a patient consumable 16 is being administered to the patient 40. A unique caregiver identifier may be included in a caregiver badge, for example.

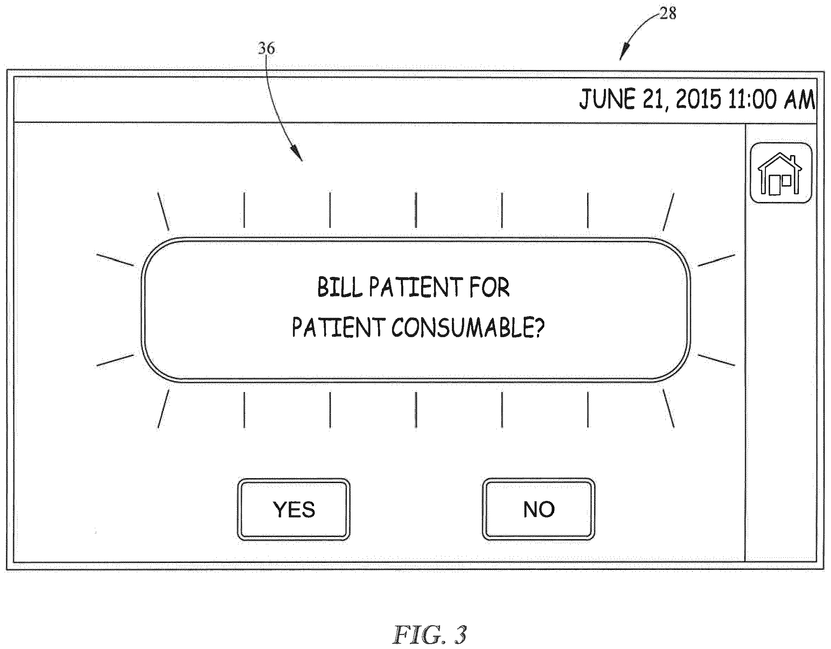

Illustratively, the user input 28 includes a touch screen display 36 having a graphical user interface as shown in FIGS. 1 and 3. The touch screen display 36 is configured to display information and/or prompts to the caregiver 38 and to receive input from the caregiver 38. In other embodiments, the user input 28 includes one or more of a keyboard, mouse, monitor, speaker, and/or any other device for commuting information between the controller 14 and the caregiver 38.

The system 10 may further include a patient consumable inventory database 34 as shown in FIG. 2. The patient consumable inventory database 34 includes patient consumable tracking information relating to each patient consumable 16 such as, but not limited to, type, quantity, location, cost, and date received. The controller 14 is configured to generate and transmit records to the patient consumable inventory database 34 via the hospital network 30 to update the database 34. In the illustrative embodiment, the controller 14 is configured to transmit instructions to the hospital network 30 to cause the hospital network 30 to update the database 34 to indicate that the patient consumable 16 has been expended.

In some embodiments, the controller 14 is in communication with peripheral devices of the patient support apparatus 12, such as peripheral device 46 shown in FIG. 2. The peripheral device 46 may be any of a number of subsystems of a patient support apparatus 12 known in the art. For example if patient support apparatus 12 is embodied as a hospital bed, the peripheral device 46 may include any one of a scale system, a side rail position monitoring system, a brake mechanism monitoring system, a bed position monitoring system, a patient position monitoring system including bed exit detection capability, or a therapy device such as a therapeutic mattress, for example. In general, the peripheral device 46 may be embodied as any subsystem or device that monitors a patient condition, monitors an operating condition of the patient support apparatus 12, controls an operating condition of the patient support apparatus 12, or provides therapy to the patient 40 supported on the patient support apparatus 12.

The hospital information system 32 may include an identifier index including identification information indicative of identified patients 40, caregivers 38, patient support apparatuses 12, and patient consumables 16. The identifier index may associate the identifiers with one another to link the identification information of patients 40, caregivers 38, patient support apparatuses 12, and patient consumables 16. The controller 14 is configured to communicate with the identifier index.

The hospital information system 32 may include patient records, caregiver records, patient support apparatus records, and patient consumable records. The patient records may include the patient's treatment history, the patient's treatment schedule, and demographic information. The patient's treatment schedule may include the date and time of future treatments and type and quantity of treatments to be administered. The caregiver records may include assignments, treatments, and therapies which have been authorized for each caregiver 38 to administer to the patient 40.

In some embodiments, the controller 14 is configured to prompt a caregiver 38 to indicate whether a patient consumable 16 being administered to the patient 40 should be billed to the patient 40 and to generate a record indicating that the patient consumable 16 should be billed to the patient 40 if the caregiver 38 responds in the affirmative to the prompt as suggested in FIG. 3. The controller 14 transmits the record to the hospital information system 32 so that the record in the hospital information system 32 may be accessed to prepare a bill for the patient 40 which includes the patient consumable 16.

The controller 14 is configured to prompt the caregiver 38 to indicate whether the patient consumable 16 is to be billed to the patient 40 in response to identifying the patient consumable 16 as suggested in FIG. 3. If the caregiver 38 indicates that the patient consumable 16 is to be billed to the patient 40, the controller 14 associates the patient consumable information with identification information of the patient 40 to generate a record that the patient consumable 16 is to be billed to the patient 40. In the illustrative embodiment, the controller14 transmits the record to the hospital information system 32 via the hospital network 30. The record in the hospital information system 32 may be accessed by a user to prepare a bill for the patient 40 which includes the patient consumable 16.

The controller 14 is configured to transmit data and instructions to the hospital network 30 to generate a record indicative that the patient consumable 16 is to be billed to the patient 40 if the caregiver 38 indicates that the patient consumable 16 is to be billed to the patient 40. If the user input 28 includes a touch screen display 36, the touch screen display 36 is configured to display a prompt for the caregiver 38 to indicate whether the patient 40 should be billed for the patient consumable 16 and further displays touch-to-accept options for the caregiver 38 to touch to input a response to the prompt as shown in FIG. 3.

In some embodiments, the controller 14 accesses the records from the hospital information system 32 and compares the identified patient consumables 16 with the patient records. The controller 14 further compares the identified caregiver 38 with the caregiver records. The controller 14 blocks unauthorized patient consumables 16 from being administered to the patient 40 and blocks unauthorized caregivers 38 from administering treatments to the patient 40.

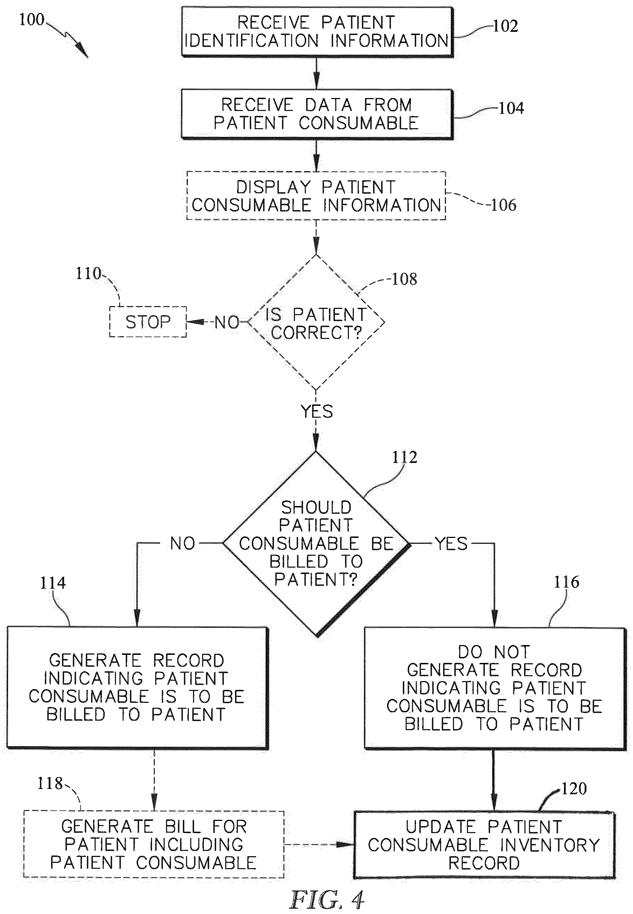

A process 100 shown in FIG. 4 provides an overview of various actions the controller 14 may take in relation to the patient consumable 16. The steps need not be performed in the order they are presented and described. Process steps shown in phantom indicate that the particular process step is optional. At an initial process step 102, the controller 14 receives unique identification information of the patient 40 associated with the patient support apparatus 12. This step may be performed when the patient 40 is first assigned to the patient support apparatus 12. In the illustrative embodiment, the unique patient identification information is transmitted to the controller 14 by the patient identifier in the patient's wristband. In other embodiments, the unique identification information of the patient 40 is received from the hospital information system 32 via the hospital network 30.

The process 100 proceeds to a step 104 where the controller 14 receives data from the patient consumable 16 as shown in FIG. 4. In the illustrative embodiment, the electro-magnetic reader 26 automatically detects a signal of the identifier 18 coupled to the patient consumable 16 in response to the identifier 18 being in the detection range 50 of the electro-magnetic reader 26 as suggested in FIG. 1. The electro-magnetic reader 26 identifies the patient consumable 16 and automatically transmits data indicative of the unique patient-consumable identification information received from the identifier 18 to the processor 22 in response to reading the identifier 18.

In an optional step 106, the controller 14 displays information relating to the patient consumable 16 on the touch screen display 36 as shown in FIG. 4. In some embodiments, an image of the patient consumable 16 is displayed on the touch screen display 36 for visual confirmation of the patient consumable 16 by the caregiver 38. In an optional step, the controller 14 prompts the caregiver 38 to confirm the patient consumable 16 is correct upon reviewing the displayed image of the patient consumable 16.

Process 100 proceeds to another optional step 108 where the controller 14 determines whether the correct patient has been identified for administration of the patient consumable 16 by accessing the patient records via the hospital network 30 as shown in FIG. 4. If, for example, the patient consumable 16 is medication, the controller 14 receives the prescription schedule via the hospital network 30 to determine whether the patient 40 is due for medication 16. An image of the patient may be displayed on the touch screen display 36 to permit the caregiver 38 to verify the patient's identity. If an incorrect patient is identified, or the patient 40 is not due for medication 16, then access to the medication 16 in a locked patient consumable container or a locked medication box is blocked as shown in step 110.

In some embodiments, if the correct patient is identified and is due for medication 16, the controller 14 receives caregiver identification information. For example, the electro-magnetic reader 26 detects a unique identification signal from a badge or RFID tag assigned to the caregiver 38. The controller 14 then determines whether the caregiver 38 is an authorized caregiver for administering medication 16 by comparing the signal received to a database of authorized caregivers available from hospital network 30. If the caregiver 38 is not authorized to administer medication 16, then access to medication 16 is blocked. If the caregiver 38 is authorized, then the controller 14 unlocks the lock 20 to allow access to the medication 16.

Optionally, the controller 14 determines whether the patient consumable 16 detected is correct by comparing the received identification signal to information received from the hospital network 30. If the patient consumable 16 is not correct, access to the patient consumable 16 is blocked. If the patient consumable 16 is correct, the controller 14 displays information related to the patient consumable 16 on the touch screen display 36. As one example, the controller 14 displays an image of a particular pill or other type of medication on the touch screen display 36 for visual confirmation of the medication 16 by the caregiver 38. Upon reviewing the displayed image, the caregiver 38 may confirm that medication 16 is correct.

Process 100 proceeds to step 112 in which the controller 14 prompts the caregiver 38 to indicate whether the patient consumable 16 should be billed to the patient 40 as shown in FIG. 4. If the caregiver 38 indicates that the patient consumable 16 should be billed to the patient 40, the controller 14 generates a record indicative that the patient consumable 16 is to be billed to the patient 40 as shown in step 114. Illustratively, the controller 14 associates the unique patient-consumable identification information with the unique identification information of the patient 40 and includes the associated information in the record. If the caregiver 38 indicates that the patient consumable 16 should not be billed to the patient 40, the controller 14 does not generate the record as shown in step 116. The controller 14 transmits the record to the hospital information system 32 so that the record may be accessed to generate a bill for the patient 40 as shown in step 118. In some embodiments, if the caregiver 38 does not input a response to the prompt, the controller 14 automatically removes the prompt and does not generate the record as shown in step 116.

If the administered patient consumable 16 is medication 16, the controller 14 optionally prompts the caregiver 38 to indicate whether or not the patient 40 digested/held down the medication 16. If medication 16 was not held down, the controller 14 does not add the medication 16 to the patient's medical record. If the medication 16 was held down, the controller 14 adds medication 16 to the patient's record including dosage amount and time of administration.

In step 120, the controller 14 generates a record indicating that the patient consumable 16 has been expended. The controller 14 transmits the record to the patient consumable inventory database 34 via the hospital network 30. The patient consumable inventory database 34 updates the inventory records to accurately reflect a number of the patient consumables 16 by type stored in the database 34.

Information may be transferred over the network 30 to the hospital information system 32 and the patient consumable inventory database 34 by the controller 14 in real time, or may be stored in the memory 24 and transferred to the hospital network 30 on an intermittent basis. In still other embodiments, when the information is stored on the controller 14, the hospital information system 32 may be operable to query the controller 14 to receive the most recent information stored by controller 14 in the memory 24. The controller 14 may combine and associate information from peripheral devices 46 as well as patient consumables 16 so that all of the information may be transferred to the hospital information system 32 as a single record. It should be understood that the network 30 may be connected to the controller 14 and the patient support apparatus 12 through a wired data link, or the network connection may be a wireless data link.

The system enables monitoring of patient consumables. Administration of consumables can be charted automatically or via prompts to caregivers so ensuring compliance with the requirement for real time recordal.

Although certain illustrative embodiments have been described in detail above, variations and modifications exist.

## Claims

1. A controller of a patient support apparatus comprising a processor configured to communicate with a hospital network, an electro-magnetic reader configured to receive an electro-magnetic signal from a patient consumable and to transmit data to the processor, and a memory having stored therein a plurality of instructions that when executed by the processor cause the controller to: receive, automatically, data from the electro-magnetic reader indicative of unique patient-consumable identification information of a patient consumable being administered to a patient associated with the patient support apparatus in response to the electro-magnetic reader receiving the electro-magnetic signal from the patient consumable, and transmit data and instructions to the hospital network to generate a record indicative that the patient consumable has been expended, the data including the associated unique patient-consumable identification information.

2. The controller of claim 1, wherein the instructions transmitted to the hospital network cause the hospital network to update an inventory database stored on the hospital network to indicate that the patient consumable has been expended.

3. The controller of either claim 1 or claim 2, wherein the plurality of instructions further cause the controller to receive unique identification information of a patient associated with the patient support apparatus from at least one of a user input and the hospital network, associate the unique patient-consumable identification information with the unique identification information of the patient, and transmit data and instructions to the hospital network to generate a record that the patient consumable has been administered to the patient, the data also including the unique identification of the patient.

4. The controller of claim 1, wherein the plurality of instructions further cause the controller to receive unique identification information of a patient associated with the patient support apparatus from at least one of a user input and the hospital network.

5. The controller of either claims 3 or claim 4, wherein the plurality of instructions further cause the controller to prompt the caregiver to verify, through the user input, unique identification information of the patient to whom the patient consumable is being administered.

6. The controller of a preceding claim, wherein the plurality of instructions further cause the controller to prompt the caregiver to indicate whether the patient consumable was digested by the patient.

7. The controller of claim 6, wherein the record indicates that the patient consumable was digested by the patient.

8. The controller of a preceding claim wherein the plurality of instructions further cause the controller to: prompt the caregiver to indicate, through a user input, whether the patient consumable is to be billed to the patient, and generate, automatically, a record indicative that the patient consumable is to be billed to the patient if the caregiver indicated that the patient consumable is to be billed to the patient.

9. The controller of claim 8, wherein the user input is a touch screen and the plurality of instructions cause the user input display (i) a first touch-to-accept option to bill the patient consumable to the patient and (ii) a second touch-to-accept option not to bill the patient consumable to the patient to prompt the caregiver to indicate whether the patient consumable is to be billed to the patient.

10. The controller of claim 9, wherein the plurality of instructions stored in the memory further cause the controller to display information relating to the patient consumable on the touch screen.

11. The controller of a preceding claim, wherein the electro-magnetic reader is configured to receive an electro-magnetic signal from the patient consumable without the patient consumable being within line-of-sight of the electro-magnetic reader.

12. The controller of any preceding claim, wherein the electro-magnetic reader includes an RFID reader configured to receive an electro-magnetic signal from an RFID tag attached to the patient consumable.

13. The controller of any one of claims 1 to 10, wherein the electro-magnetic reader includes a barcode reader configured to receive an electro-magnetic signal from a barcode attached to the patient consumable.

14. The controller of a preceding claim including a user input configured to transmit a signal to the processor responsive to an activation of the user input by a caregiver.
